# EUROPEAN PATENT SPECIFICATION

(11) **EP 1 414 513 B1**
(45) Date of publication and mention of the grant of the patent: **01.04.2009**
(21) Application number: 01986082.4
(22) Date of filing: 02.11.2001
(51) Int. Cl.: A61M 25/10, A61M 27/00

(54) **CATHETER FOR REMOVAL OF SOLIDS FROM SURGICAL DRAINS**
KATHETER ZUR ENTFERNUNG VON FESTSTOFFEN AUS CHIRURGISCHEN DRAINS
SONDE SERVANT A ELIMINER DES PARTICULES SOLIDES DE DRAINS CHIRURGICAUX

(30) Priority: 03.11.2000 US 245796 P
(43) Date of publication of application: 06.05.2004
(73) Proprietor: THE CLEVELAND CLINIC FOUNDATION, Cleveland, OH 44195 (US)
(72) Inventor: LIDDICOAT, John, Boston, MA 02215 (US); GILLINOV, Alan, Marc, Orange Village, OH 44022 (US); GOODIN, Mark, S., Solon, OH 44139 (US); BYERMAN, Bryan, South Euclid, OH 44121 (US)
(74) Representative: Roos, Peter
(86) International application number: PCT/US2001/045648
(87) International publication number: WO 2002/038198

(56) References cited:
- US-A- 4 148 319
- US-A- 4 228 802
- US-A- 4 698 058
- US-A- 5 370 610
- US-A- 5 522 801

## Description

### BACKGROUND OF THE INVENTION

### Field of the Invention

This invention relates to a catheter for removing solid or semi-solid material from surgical drains. More particularly, it relates to a catheter assembly designed to clear surgical drains of clotted blood and particulate matter.

### Description of Related Art

At the conclusion of many surgical procedures, drains are left in the patient to prevent accumulation of blood and other fluids. These drains are generally attached to a suction apparatus, facilitating removal of unwanted fluids. When drains are used to remove blood, these drains have a propensity to become occluded by blood clots, and the drains cease to function.

Clotting of chest drains is a particular problem in cardiac surgery. Because cardiac surgical patients receive large doses of anticoagulants during surgery and develop platelet dysfunction, most or all such patients bleed several hundred milliliters in the first 24 postoperative hours. In order to evacuate this blood, patients receive 2 to 4 chest tubes. Blood tends to clot in the chest tubes, and nurses attempt to 'strip' the drains to ensure their continued function. Unfortunately, such efforts are frequently unsuccessful. When a patient's chest tubes cease to function by becoming clogged or obstructed by clotted blood or other particulate.matter, clotted blood may collect around the heart creating the life-threatening condition of cardiac tamponade. Up to 5% of cardiac surgical patients develop this important complication.

There is a need in the art for a device that can be used to clear surgical drains of clotted blood and thereby maintain their function. Such a device would be of great use, particularly in chest tubes for cardiac surgical patients. Preferably, such a device would function as an integral part of a surgical drain assembly (such as a chest tube assembly) in order to maintain a closed system and ensure sterility. Preferably, such a device could also be used to clear other types of surgical drains, including, e.g. biliary catheters and empyema tubes, of unwanted and compromising solid debris.

US 5 370 610 discloses a device according to the preamble of claim 1.

### SUMMARY OF THE INVENTION

A catheter for removing solid or semi-solid material from a surgical drain is provided as defined in claim 1. The catheter has a proximal region and a distal region, and a solids removal member disposed within its distal region. The distal region is suitable for insertion into a surgical drain to deliver the solids removal member within the surgical drain near the distal end of the drain.

A catheter assembly is also provided for removing solid or semi-solid material from a surgical drain. The assembly has a catheter having a proximal region and a distal region, a flexible protective sleeve having a first end and a second end, an aspiration port, and an aspiration vacuum port. The first end of the protective sleeve is attached to the catheter in the proximal region thereof. The sleeve is adapted to permit the distal region of the catheter to be extended into and withdrawn from the surgical drain. The aspiration port is located in the distal region of the catheter. The aspiration vacuum port is located in the proximal region of the catheter. The aspiration port and aspiration vacuum port are connected via an aspiration conduit. The distal region of the catheter is suitable for insertion into a surgical drain.

### BRIEF DESCRIPTION OF THE DRAWINGS

Fig. 1 is a side view of a catheterized surgical drain assembly having a catheter according to the invention.
Fig. 2 is a side view of the invented catheterized surgical drain assembly with the catheter in a retracted position.
Fig. 3 is a side view as in Fig. 2, except that the catheter is in an extended position.
Fig. 4 is a perspective view of an invented catheter having an inflatable balloon according to a first preferred embodiment of the invention.
Fig. 5 is a cross-sectional view taken along line 5-5 of Fig. 4.
Fig. 6 is a side view, partially in section, of a catheterized surgical drain assembly having the invented catheter of Fig. 4, shown prior to the inflatable balloon penetrating a blood clot in the surgical drain.
Fig. 7 is a side view as in Fig. 6, shown after penetration of the blood clot by the inflatable balloon.
Fig. 8 is a schematic view of the distal region of a surgical drain with an invented catheter having a deployable umbrella member according to a second preferred embodiment of the invention.
Fig. 9 is a schematic view of the distal region of a surgical drain with an invented catheter having a deployable spring according to a third preferred embodiment of the invention.
Fig. 10 is a schematic view of the distal region of a surgical drain with an invented catheter having a slicer according to a fourth preferred embodiment of the invention.
Fig. 11 is a cross-sectional view of the slicer taken along line 11-11 in Fig. 10.
Fig. 12 is a schematic view of the distal region of a surgical drain with an invented catheter having a fixed spring according to a fifth preferred embodiment of the invention.

### DETAILED DESCRIPTION OF THE PREFERRED EMBODIMENTS OF THE INVENTION

In the description that follows, when a range such as 5 to 25 (or 5-25) is given, this means preferably at least 5 and, separately and independently, preferably not more than 25.

As used herein, the terms proximal and distal are generally construed with reference to a patient that has been fitted with a surgical drain. For example, the distal end of a surgical drain (or distal region of a catheter) is that end (or region) which is nearer or adjacent to the patient. Conversely, the proximal end of a surgical drain (or proximal region of a catheter) is that end (or region) which is further from the patient. Likewise, a distal element (or the distal side of an element) is nearer to the patient than a proximal element (or the proximal side of an element).

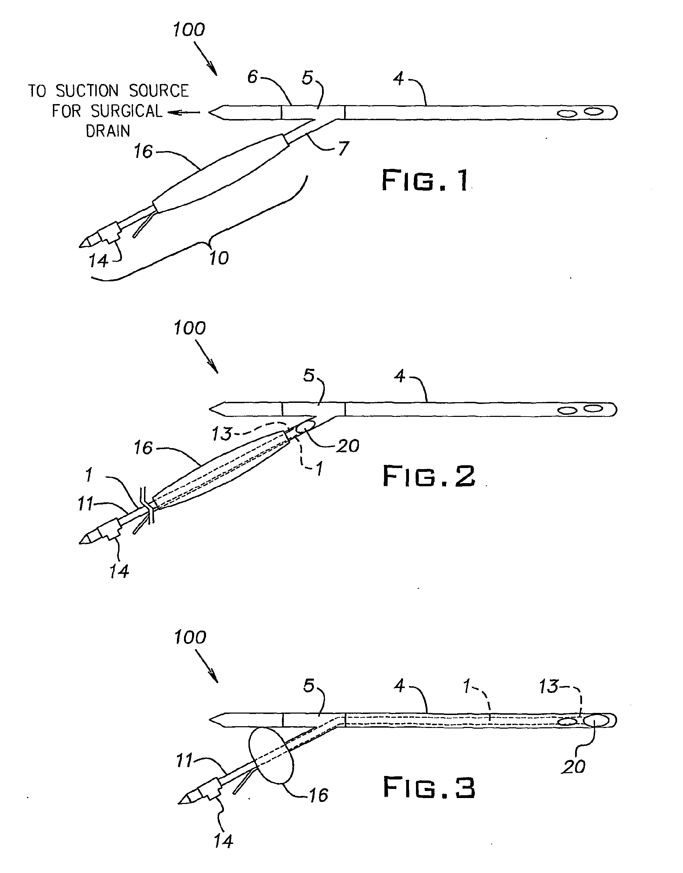

Fig. 1 shows a catheterized surgical drain assembly 100 according to the invention. The assembly 100 has a surgical drain 4 (such as a chest tube, biliary catheter, empyema tube, or other surgical drain), and a catheter assembly 10. The catheter assembly 10 is coupled to the surgical drain 4 via a catheter adaptor 5 having a suction lumen 6 and a catheter lumen 7. Preferably, the adaptor 5 has a one-way valve or check valve to prevent air or solid matter from proceeding through the adaptor 5 and into the drain 4 toward a patient. The adaptor 5 is preferably a y-adaptor as shown in Fig. 1, less preferably a tee-adaptor, less preferably some other configuration known in the art. A y-adaptor is preferred because it guides the insertion of catheter 1 into the drain 2 when the catheter 1 is in the extended position (explained below), without obstructing the suction path between the surgical drain 4 and the suction lumen 7 when in the retracted position (explained below).

The catheter assembly 10 has a catheter 1, a protective sleeve 16, and a solids removal member 20. In a preferred embodiment, the catheter assembly 10 is provided as an integrated catheter assembly with all the above components pre-assembled. In another preferred embodiment, the catheter assembly 10 is provided together with the catheter adaptor 5 and surgical drain 4 as an integrated catheterized surgical drain assembly. Preferably, the protective sleeve 16 is in the form of a flexible or expandable or elastic sheath having first and second ends. Preferably, the first end of the sheath is connected to the catheter 1 in proximal region 11, and its second end connected to the adaptor 5 (catheter lumen 7). The protective sleeve 16 provides a closed system for the catheterized surgical drain assembly 100, thereby ensuring a sterile environment within the assembly as the catheter is shifted between its retracted and extended positions as explained in the next paragraph. The protective sleeve 16 allows the catheter 1 to be reused without compromising the sterility of the system, and also preferably prevents exposure to secreted bodily fluids by healthcare personnel. Protective sleeve 16 is made from a flexible material, preferably a plastic or rubber material, e.g. latex, less preferably polypropylene or polyethylene, less preferably polytetrafluoroethylene, less preferably neoprene rubber, silicone or silicone rubber, less preferably ethylene propylene diene monomer (EPDM), less preferably any other suitable flexible material. Optionally, the protective sleeve 16 is provided in an accordion pattern such that it neatly expands and contracts as the catheter 1 is extended and retracted into/from the drain 4. The catheter 1 is made as customarily known in the art, from known or conventional materials.

Referring to Figs. 2 and 3, an invented catheterized surgical drain assembly is shown with the catheter 1 in a retracted position and in an extended position respectively. As seen in Fig. 2, when the catheter 1 is in a retracted position, the catheter is retracted into the catheter lumen 7 of the adaptor 5, and does not obstruct the passageway of the surgical drain 4, or the path from the surgical drain 4 to the suction lumen 6. As seen in Fig. 3, when in the extended position, the catheter 1 extends through the catheter adaptor 5 (preferably a y-adaptor) into the surgical drain 4.

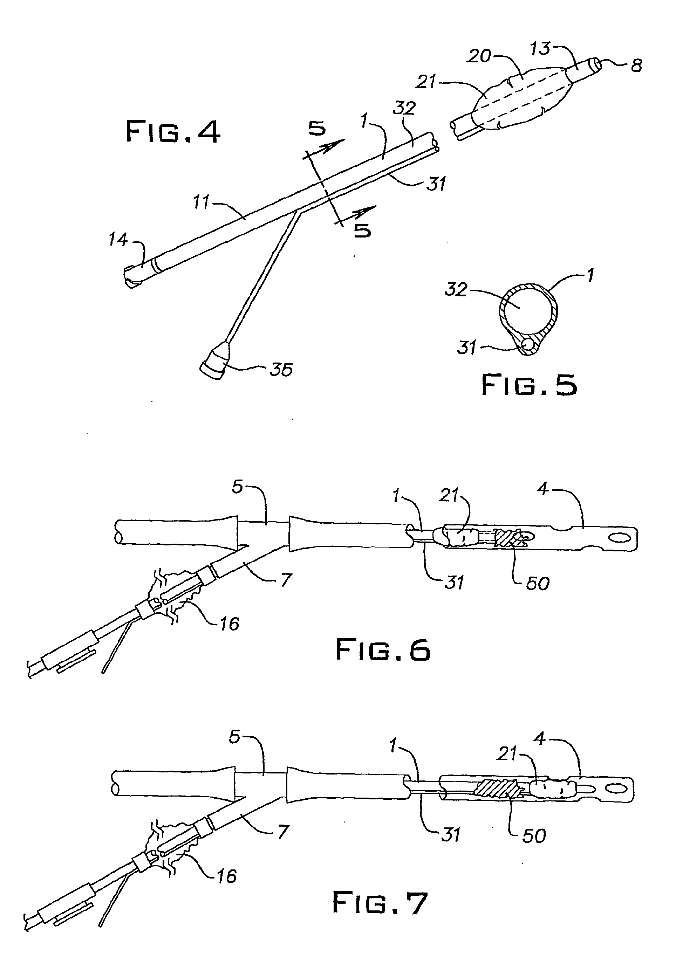

The catheter 1 has a proximal region 11 and a distal region 13. As can be seen in Fig. 4, the invented catheter 1 preferably has in its distal region 13 a solids removal member 20,and an aspiration port 8. The solids removal member 20 is advanced into the bore of the surgical drain 4 when the catheter 1 is in its extended position. The aspiration port 8 is preferably connected via an aspiration conduit 32 to an aspiration vacuum port 14 in the proximal region 11 of the catheter 1. The vacuum port 14 can be capped or sealed when not in use to maintain sterility and vacuum in the surgical drain 4.

The catheter can be used to clear a surgical drain, such as a chest tube, of solid, semi-solid and liquid material by two mechanisms. Once the catheter is fully extended into the drain 4, suction may be used to aspirate material into the hollow bore or aspiration conduit 32 of the catheter 1. Intermittent occlusion of the aspiration vacuum port 14 in the proximal region 11 of the catheter causes intermittent suction at its distal aspiration port 8. In addition, actuation of the solids removal member 20 followed by withdrawal of the catheter 1 will clear the surgical drain 4 of larger collections of material and material adherent to the sides of the surgical drain 4. Aspiration is preferably achieved by connecting a suction source (preferably separate from that for the surgical drain 4) to the vacuum port 14 to aspirate solid, semi-solid and/or particulate material out of the surgical drain 4 through the aspiration port 8 of the catheter. The solids removal member 20 is actuated by an appropriate actuation means (as described herein or known in the art) that can be provided via an actuation conduit 31 between an actuation port 35 in the proximal region 11 of the catheter 1, and the solids removal member 20. Preferably, the actuation conduit 31 is separate from the aspiration conduit 32. (See Fig. 5). The catheter 1 may be used to clear the surgical drain 4 of debris by employing suction, withdrawal by the solids removal member 20, or both. In each of the following preferred embodiments, the catheter 1 can have (though does not require) an aspiration port 8 for aspiration of solid matter.

Referring to Fig. 4, the solids removal member 20 is an inflatable balloon 21 according to a first preferred embodiment of the invention. In this embodiment, the inflatable balloon 21 is inflated once the catheter 1 is in the fully extended position. Then the catheter 1 is withdrawn from the drain 4, removing blood clots and particulate matter too large to be aspirated into aspiration port 8, or that were stuck to the inner wall of the surgical drain 4. The balloon 21 is inflated by an appropriate inflation fluid, preferably air or saline, that is preferably injected into the actuation port 35 and delivered to the balloon 21 via the actuation conduit 31. Preferably, actuation port 35 is adapted to mate with a standard syringe for ease of balloon inflation.

A catheter 1 equipped with an inflatable balloon 21 as described is typically used in the following manner. (Though the following method is provided with reference to an inflatable balloon 21, it will be understood that the method is generally applicable to an invented catheter having a solids removal member other than an inflatable balloon). Once a caregiver or healthcare professional notes an apparent obstruction in a surgical drainage tube (i.e. indicated by cessation of movement of fluid through the drain 4, or collapsing of the tubing connecting the drain 4 to the collection unit), the caregiver first ensures the balloon 21 is uninflated (i.e. is in a collapsed position). If the catheter 1 is equipped with an aspiration port 8, the vacuum port 14 is sterilely attached to a suction source. In this manner, a continuous vacuum is applied to the aspiration port 8 to assist in eliminating thrombi 50 and particulate from the surgical drain 4. Continuous aspiration may aid in tunneling through a clot or thrombus 50 in order for the catheter 1 to penetrate the thrombus 50 and deliver the inflatable balloon 21 to the distal side of the thrombus 50. (See Figs. 6-7).

Once the vacuum has been applied, the catheter 1 is slowly inserted into the surgical drain 4 until it approaches the distal end of the drain 4. Next, the balloon 21 is actuated or inflated such that the balloon 21 engages and pushes against the inner wall surface of the drain 4. Once the balloon is inflated, the catheter 1 (and thereby inflated balloon 21) is slowly pulled back or withdrawn to the proximal end of the drain 4, with the inflated balloon 21 dislodging and pulling any particulate or thrombi 50 with it. Once such solids are dislodged from the inner wall of the drain 4, the solids are evacuated from the drain, either through the aspiration port 8 in the catheter 1, or through the suction lumen 6 by the surgical drain suction source. Following the above procedure, the balloon is de-actuated (i.e. deflated to a collapsed position) and the catheter 1 is withdrawn back into a retracted position within the catheter lumen 7. Once the catheter 1 is fully retracted, normal operation of the surgical drain 4 is resumed.

The above procedure could be performed at regular intervals (i.e. every 1, 2, 3, 4, 5, 6, 7, 8, 9, 10, 12, 24, 36, or 72, hours), or upon discovery of an occluded surgical drain 4. For surgical drains having very small diameters (i.e. 1-5, 1-3, or 1-2, mm) such as those employed for minimally invasive surgeries, the drain 4 is preferably cleaned via the above procedure at shorter intervals, preferably every 0.5-24, preferably 0.5-12, preferably 0.5-6, preferably 0.6-4, preferably 0.8-2, preferably about 1, hour(s).

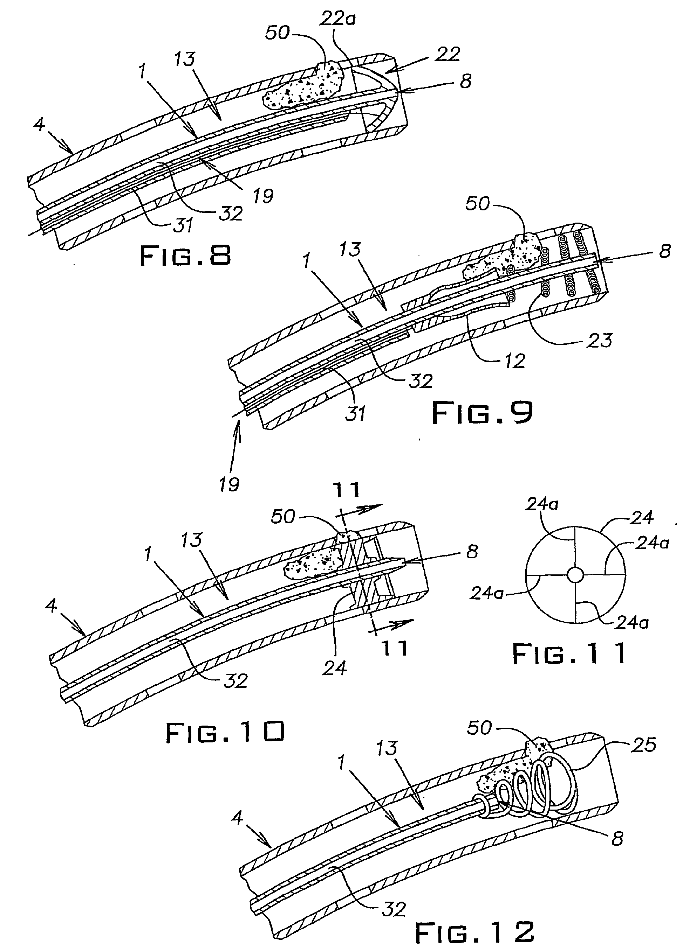

Referring to Fig. 8, the solids removal member 20 is a deployable umbrella member 22 according to a second preferred embodiment of the invention. In this embodiment, the distal region 13 of the catheter 1 has a remotely deployable umbrella member 22. The catheter 1 is introduced into the surgical drain 4 with the umbrella member 22 in a collapsed position. Once the catheter 1 is fully extended with its tip near the distal end of the surgical drain 4, the umbrella member 22 is deployed into an open position as shown in Fig. 8. A preferred means of deploying the umbrella member is a guide wire 19 as known in the art. Guide wire 19 is attached at one end to the umbrella member 22 as shown in Fig. 8. The guide wire is threaded through the actuation conduit 31 to exit the actuation port 35 in the proximal region of the catheter where it can be manipulated by a caregiver to actuate the umbrella member 22. Less preferably, umbrella member 22 can be actuated via other known or conventional means.

When in the open position, the terminal edge 22a of umbrella member 22 preferably engages the inner wall of the drain 4. Similarly as described above with respect to the first preferred embodiment, the catheter 1 (and umbrella member 22) is slowly withdrawn from the drain 4, with the umbrella member 22 dislodging and pulling occluding thrombi 50 and particulate matter from the drain 4. Also, as in the first preferred embodiment, an aspiration port 8 can be provided, and continuous suction applied to aid clearing of solid or particulate matter from the surgical drain 4, and delivery of the umbrella member 22 to the distal side of any present thrombi 50.

Referring to Fig. 9, the solids removal member 20 is a deployable spring 23 according to a third preferred embodiment of the invention. This embodiment is used in a similar manner to the deployable umbrella member embodiment previously described. The distal region 13 of the catheter 1 has a remotely deployable coiled spring 23. The catheter 1 is introduced into the surgical drain 4 with the spring 23 in a collapsed position enclosed in sheath 12. The sheath 12 is slidably engaged to the outer surface of the catheter 1, and causes the spring to collapse inward when the spring is pulled within the sheath 12. Once the catheter has been fully extended as previously described, the sheath 12 is actuated (i.e. retracted) causing the coiled spring 23 to deploy as shown in Fig. 9. When deployed, spring 23 preferably engages the inner surface of the surgical drain 4.

Preferably, the sheath 12 is actuated by a guide wire 19 similarly as described above. The spring 23 is captured within the sheath 12 by pushing the guide wire 19 through the actuation conduit 31 while holding the catheter 1 in place to prevent the advance of the spring 23. Conversely, the spring 23 is deployed by pulling the guide wire 19 in the direction of the proximal region 11 and away from the distal region 13 while holding the catheter 1 in place. With the coiled spring 23 deployed, the caregiver or healthcare professional removes thrombi 50 and particulate by slowly withdrawing the catheter 1, in the manner previously described.

Referring to Fig. 10, the solids removal member 20 is a slicer 24 according to a fourth preferred embodiment of the invention. The slicer 24 preferably has a circular cross-section as shown in Fig. 11, with both proximal and distal cutting edges. Preferably, the slicer 24 has a plurality of radial slats 24a as shown in Fig. 11, preferably at least 2 slats, more preferably 4 slats, each slat also having proximal and distal cutting edges. The slicer 24 is fixedly engaged to the exterior surface of the catheter 1. The slicer 24 has a slightly smaller diameter than the inner diameter of the surgical drain 4. Such slightly smaller diameter allows the slicer 24 to clear thrombi 50 and other debris while allowing translation of the slicer 24 along the drain 4. The slicer functions by inserting the catheter 1 into the surgical drain 4 to deliver the slicer to the distal end of the drain 4. Next, the catheter 1 is slowly withdrawn through the drain 4 such that the cutting edges of the slicer 24 cut up and dislodge entrained thrombi and particulate to be evacuated as previously described.

Referring to Fig. 12, the solids removal member 20 is a fixed spring 25 attached in the distal region 13 of the catheter 1 according to a fifth preferred embodiment of the invention. Preferably, the fixed spring 25 has a helical pattern, e.g. as shown in Fig. 12. The fixed spring 25 can be a coiled spring, which is then subsequently coiled into a helical pattern, or it can be an uncoiled strip of material (preferably metal) that has been oriented in a helical pattern. The catheter 1 having such a fixed spring 25 can be inserted into a surgical drain as previously described, and preferably is rotated such that the fixed spring 25 contacts and breaks up entrained thrombi 50 or other particulate. Next, the catheter 1 is withdrawn from the surgical drain 4, and the dislodged solids are evacuated either through aspiration port 8 in the catheter 1, or through the suction lumen 6 by the surgical drain suction source.

Although the hereinabove described embodiments of the invention constitute the preferred embodiments, it should be understood that modifications can be made thereto without departing from the scope of the invention as set forth in the appended claims.

## Claims

1. A device for removing solid or semi-solid material from a surgical drain (4), the device comprising a catheter (1) having a proximal region (11) and a distal region (13), said distal region (13) being suitable for insertion into a surgical drain (4),
**characterized in that** the device also comprises a solids removal member (20) distinct from and attached to said distal region (13) of said catheter (1), wherein said distal region (13) delivers said solids removal member (20) within said surgical drain (4), and **in that** said solids removal member (20) is actuable after insertion into said surgical drain (4) so that it is effective to remove debris from the surgical drain (4) on withdrawal therefrom.

2. A device according to claim 1, wherein the solids removal member (20) is selected from an inflatable balloon (21), a deployable umbrella member (22), a deployable spring (23), a slicer (24) having proximal and distal cutting edges, and a fixed spring (25), said fixed spring (25) being either an uncoiled strip of material oriented in a helical pattern, or a coiled spring.

3. A device according to claim 1 or 2, further comprising a flexible protective sleeve (16) having a first end and a second end, wherein said first end of said protective sleeve (16) is attached to said catheter (1) in said proximal region (11) thereof, said sleeve (16) being adapted to permit the distal region (13) of said catheter (1) to be extended into and withdrawn from said surgical drain (4).

4. A device according to claim 3, wherein said protective sleeve (16) is effective to maintain a sterile environment within said surgical drain (4), and to prevent exposure to secreted bodily fluids by healthcare personnel.

5. A device according to any one of claims 1-4, further comprising an aspiration port (8) located in the distal region (13) of said catheter (1), and an aspiration vacuum port (14) located in the proximal region (11) of said catheter (1), wherein said aspiration port (8) and said aspiration vacuum port (14) are connected via an aspiration conduit (32).

6. A device according to any one of claims 1-5, further comprising an actuation port (35) in the proximal region (11) thereof, wherein said solids removal member (20) is actuatable by an actuation means via an actuation conduit (31) between said actuation port (35) and said solids removal member (20).

7. A device according to claim 3, further comprising a catheter adaptor (5), wherein said second end of said protective sleeve (16) is attached to said catheter adaptor (5).

8. A device according to claim 7, wherein said catheter adaptor (5) is a y-adaptor having a catheter lumen (7) and a suction lumen (6), said second end of said protective sleeve (16) being attached to said catheter lumen (7) of said adaptor (5), and wherein said suction lumen (6) and said surgical drain (4) define a suction pathway for said surgical drain (4).

9. A device according to claim 8, wherein said catheter (1) does not obstruct said suction pathway when said catheter (1) is in a retracted position.

10. A device according to any one of claims 3, 4 and 7-9, wherein said catheter (1), solids removal member (20) and protective sleeve (16) are provided together with a catheter adaptor (5) and said surgical drain (4) as an integrated catheterized surgical drain assembly (100).

11. A device according to any one of claims 1-10, wherein said solids removal member (20) is an inflatable balloon (21).

12. A device according to claim 11, further comprising an actuation port (35) in the proximal region (11) of said catheter (1), and an actuation conduit (31) between said actuation port (35) and said balloon (21), wherein said balloon (21) is inflatable via an inflation fluid that is delivered to said actuation port (35).

13. A device according to any one of claims 1-10, wherein said solids removal member (20) is a deployable umbrella member (22).

14. A device according to any one of claims 1-10, wherein said solids removal member (20) is a deployable spring (23).

15. A device according to claim 14, further comprising a sheath (12) which is slidably engaged to said catheter (1), wherein said sheath (12) a) is adapted to enclose said deployable spring (23) to retain said spring in a collapsed position thereof, and b) is retractable from said spring (23) via an actuation means causing said spring (23) to deploy.

16. A device according to any one of claims 1-10, wherein said solids removal member (20) is a slicer (24) having proximal and distal cutting edges.

17. A device according to any one of claims 1-10, wherein said solids removal member (20) is a fixed spring (25), said fixed spring (25) being either an uncoiled strip of material oriented in a helical pattern, or a coiled spring.

## Patentansprüche

1. Vorrichtung zum Beseitigen eines festen oder halbfesten Materials von einem chirurgischen Abfluss (4), wobei die Vorrichtung einen Katheter (1) mit einem nahen Bereich (11) und einem entfernten Bereich (13) umfasst, wobei der entfernte Bereich (13) geeignet ist für eine Einführung in einen chirurgischen Abfluss (4),
**dadurch gekennzeichnet, dass** die Vorrichtung auch ein Element (20) zur Beseitigung von Feststoffen umfasst, das sich von dem entfernten Bereich (13) des Katheters (1) unterscheidet und an diesem befestigt ist, wobei der entfernte Bereich (13) das Element (20) zur Beseitigung von Feststoffen innerhalb des chirurgischen Abflusses (4) übergibt, und **dadurch gekennzeichnet, dass** das Element (20) zur Beseitigung von Feststoffen nach der Einführung in den chirurgischen Abfluss (4) betätigbar ist, so dass es wirksam ist, um Ablagerungen (bzw. Bruchstücke) aus dem chirurgischen Abfluss (4) beim Zurückziehen daraus zu entfernen.

2. Vorrichtung nach Anspruch 1 wobei das Element (20) zur Beseitigung von Feststoffen ausgewählt ist aus einem aufblasbaren Ballon (21), einem entfaltbaren Schirmelement (22), einer entfaltbaren (bzw. entspannbaren) Feder (23), einer Schneidvorrichtung (24) mit nahen und entfernten Schneidkanten und einer befestigten Feder (25), wobei die befestigte Feder (25) entweder aus einem abgewickelten Materialstreifen besteht, der in einem spiralenförmigen Muster orientiert ist, oder aus einer Schraubenfeder.

3. Vorrichtung nach Anspruch 1 oder 2, die ferner eine flexible Schutzhülse (16) mit einem ersten Ende und einem zweiten Ende umfasst, wobei das erste Ende der Schutzhülse (16) an dem Katheter (1) in dem nahen Bereich (11) desselben befestigt ist, wobei die Hülse (16) so angepasst ist, dass es dem entfernten Bereich (13) des Katheters (1) möglich ist, sich in den chirurgischen Abfluss (4) hineinzuerstrecken und aus dem chirurgischen Abfluss (4) wieder herausgezogen zu werden.

4. Vorrichtung nach Anspruch 3, wobei die Schutzhülse (16) wirksam ist, um eine sterile Umgebung innerhalb des chirurgischen Abflusses (4) aufrechtzuerhalten und um ein Ausgesetztsein gegenüber abgesonderten körperlichen Flüssigkeiten durch das Personal des Pflege-/Gesundheitswesens zu verhindern.

5. Vorrichtung nach einem der Ansprüche 1 bis 4, die ferner eine Ansaugöffnung (8) umfasst, die sich in dem entfernten Bereich (13) des Katheters (1) befindet, und eine Ansaugvakuumöffnung (14), die sich in dem nahen Bereich (11) des Katheters (1) befindet, wobei die Ansaugöffnung (8) und die Ansaugvakuumöffnung (14) über einen Ansaugkanal (32) miteinander verbunden sind.

6. Vorrichtung nach einem der Ansprüche 1 bis 5, die ferner eine Betätigungsöffnung (35) in dem nahen Bereich (11) desselben umfasst, wobei das Element (20) zur Beseitigung von Feststoffen durch ein Betätigungsmittel über einen Betätigungskanal (31) zwischen der Betätigungsöffnung (35) und dem Element (20) zur Beseitigung von Feststoffen betätigbar ist.

7. Vorrichtung nach Anspruch 3, die ferner einen Katheteradapter (5) umfasst, wobei das zweite Ende der Schutzhülse (16) an dem Katheteradapter (5) befestigt ist.

8. Vorrichtung nach Anspruch 7, wobei der Katheteradapter (5) ein y-Adapter mit einem Katheterlumen (7) und einem Sauglumen (6) ist, wobei das zweite Ende der Schutzhülse (16) an dem Katheterlumen (7) des Adapters (5) befestigt ist, und wobei das Sauglumen (6) und der chirurgische Abfluss (4) eine Saugbahn für den chirurgischen Abfluss (4) definieren.

9. Vorrichtung nach Anspruch 8, wobei der Katheter (1) die Saugbahn nicht behindert, wenn der Katheter (1) sich in einer eingezogenen Position befindet.

10. Vorrichtung nach einem der Ansprüche 3, 4 und 7 bis 9, wobei der Katheter (1), das Element (20) zur Beseitigung von Feststoffen (20) und die Schutzhülse (16) zusammen mit einem Katheteradapter (5) und dem chirurgischen Abfluss (4) als ein integrierter, katheterisierter, chirurgischer Abflusszusammenbau (100) bereitgestellt sind.

11. Vorrichtung nach einem der Ansprüche 1 bis 10, wobei das Element (20) zur Beseitigung von Feststoffen ein aufblasbarer Ballon (21) ist.

12. Vorrichtung nach Anspruch 11, die ferner eine Betätigungsöffnung (35) in dem nahen Bereich (11) des Katheters (1) und einen Betätigungskanal (31) zwischen der Betätigungsöffnung (35) und dem Ballon (21) umfasst, wobei der Ballon (21) über ein Fluid zum Aufblasen aufblasbar ist, das der Betätigungsöffnung (35) zugeführt wird.

13. Vorrichtung nach einem der Ansprüche 1 bis 10, wobei das Element (20) zur Beseitigung von Feststoffen ein entfaltbares Schirmelement (22) ist.

14. Vorrichtung nach einem der Ansprüche 1 bis 10, wobei das Element (20) zur Beseitigung von Feststoffen eine entfaltbare Feder (23) ist.

15. Vorrichtung nach Anspruch 14, die ferner eine Hülle (12) umfasst, die mit dem Katheter (1) verschiebbar in Eingriff gebracht ist, wobei die Hülle (12) a) so angepasst ist, dass sie die entfaltbare Feder (23) einschließt, um so die Feder in einer zusammengeklappten (bzw. gespannten) Position derselben zu behalten, und b) von der Feder (23) über ein Betätigungsmittel zurückziehbar ist, das die Feder (23) veranlasst, sich zu entfalten.

16. Vorrichtung nach einem der Ansprüche 1 bis 10, wobei das Element (20) zur Beseitigung von Feststoffen eine Schneidvorrichtung (24) mit nahen und entfernten Schneidkanten aufweist.

17. Vorrichtung nach einem der Ansprüche 1 bis 10, wobei das Element (20) zur Beseitigung von Feststoffen eine befestigte Feder (25) ist, wobei die befestigte Feder (25) entweder aus einem abgewickelten Materialstreifen besteht, der in einem spiralenförmigen Muster orientiert ist, oder aus einer Schraubenfeder.

## Revendications

1. Dispositif permettant d'enlever de la matière solide ou semi-solide d'un drain chirurgical (4), ce dispositif comportant un cathéter (1) ayant une zone proximale (11) et une zone distale (13), ladite zone distale (13) étant apte à être insérée dans un drain chirurgical (4),
**caractérisé en ce que** le dispositif comprend aussi un élément d'enlèvement de solides (20) distinct de et rattaché à ladite zone distale (13) dudit cathéter (1), ladite zone distale (13) délivrant ledit élément d'enlèvement de solides (20) dans ledit drain chirurgical (4), et **en ce que** ledit élément d'enlèvement de solides (20) est actionnable après insertion dans ledit drain chirurgical (4), de sorte qu'il est efficace pour enlever les débris se trouvant dans le drain chirurgical (4) lorsqu'on le retire de ce dernier.

2. Dispositif selon la revendication 1, dans lequel l'élément d'enlèvement de solides (20) est sélectionné parmi un ballon gonflable (21), un élément en parapluie déployable (22), un ressort déployable (23), une trancheuse (24) ayant des arêtes de coupe proximale et distale et un ressort fixe (25), ledit ressort fixe (25) étant soit une bande déroulée de matériau orientée suivant un motif d'hélice, soit un ressort enroulé.

3. Dispositif selon la revendication 1 ou 2, comprenant en outre un manchon protecteur souple (16) ayant une première et une seconde extrémité, ladite première extrémité dudit manchon protecteur (16) étant rattachée audit cathéter (1) dans ladite zone proximale de celui-ci (11), ledit manchon (16) étant apte à permettre à la zone distale (13) dudit cathéter (1) d'être étendue dans et retirée dudit drain chirurgical (4).

4. Dispositif selon la revendication 3, ledit manchon protecteur (16) étant efficace pour maintenir un environnement stérile dans ledit drain chirurgical (4) et pour prévenir l'exposition du personnel de soins aux fluides corporels secrétés.

5. Dispositif selon l'une quelconque des revendications 1 à 4, comprenant en outre un port d'aspiration (8) localisé dans la zone distale (13) dudit cathéter (1) et un port de vide d'aspiration (14) localisé dans la zone proximale (11) dudit cathéter (1), ledit port d'aspiration (8) et ledit port de vide d'aspiration (14) étant connectés via une conduite d'aspiration (32).

6. Dispositif selon l'une quelconque des revendications 1 à 5, comprenant en outre un port d'actionnement (35) dans sa zone proximale (11), ledit élément d'enlèvement de solides (20) pouvant être actionné par un moyen d'actionnement via une conduite d'actionnement (31) entre ledit port d'actionnement (35) et ledit élément d'enlèvement de solides (20).

7. Dispositif selon la revendication 3, comprenant en outre un adaptateur de cathéter (5), ladite seconde extrémité dudit manchon protecteur (16) étant rattachée audit adaptateur de cathéter (5).

8. Dispositif selon la revendication 7, ledit adaptateur de cathéter (5) étant un adaptateur en y ayant un lumen de cathéter (7) et un lumen de succion (6), ladite seconde extrémité dudit manchon protecteur (16) étant rattachée audit lumen de cathéter (7) dudit adaptateur (5) et ledit lumen de succion (6) et ledit drain chirurgical (4) définissant un passage de succion pour ledit drain chirurgical (4).

9. Dispositif selon la revendication 8, ledit cathéter (1) n'obstruant pas ledit passage de succion lorsque ledit cathéter (1) est en position rétractée.

10. Dispositif selon l'une quelconque des revendications 3, 4 et 7 à 9, lesdits cathéter (1), élément d'enlèvement de solides (20) et manchon protecteur (16) étant équipés ensemble d'un adaptateur de cathéter (5) et dudit drain chirurgical (4) sous forme d'assemblage de drain chirurgical cathétérisé intégré (100).

11. Dispositif selon l'une quelconque des revendications 1 à 10, ledit élément d'enlèvement de solides (20) étant un ballon gonflable (21).

12. Dispositif selon la revendication 11, comprenant en outre un port d'actionnement (35) dans la zone proximale (11) dudit cathéter (1) et une conduite d'actionnement (31) entre ledit port d'actionnement (35) et ledit ballon (21), ledit ballon (21) étant gonflable via un fluide de gonflage qui est délivré audit port d'actionnement (35).

13. Dispositif selon l'une quelconque des revendications 1 à 10, ledit élément d'enlèvement de solides (20) étant déployable à la manière d'un parapluie (22).

14. Dispositif selon l'une quelconque des revendications 1 à 10, ledit élément d'enlèvement de solides (20) étant un ressort déployable (23).

15. Dispositif selon la revendication 14, comprenant en outre une gaine (12) qui s'engage en coulissant dans ledit cathéter (1), ladite gaine (12) a) étant apte à entourer ledit ressort déployable (23) pour retenir ledit ressort dans sa position écrasée et b) étant rétractable depuis ledit ressort (23) via un moyen d'actionnement provoquant le déploiement dudit ressort (23).

16. Dispositif selon l'une quelconque des revendications 1 à 10, ledit élément d'enlèvement de solides (20) étant une trancheuse (24) ayant des arêtes de coupe proximale et distale.

17. Dispositif selon l'une quelconque des revendications 1 à 10, ledit élément d'enlèvement de solides (20) étant un ressort fixe (25), ledit ressort fixe (25) étant soit une bande déroulée de matériau orientée suivant un modèle d'hélice, soit un ressort enroulé.
